# EUROPÄISCHE PATENTANMELDUNG

(11) **EP 0 589 357 A2**
(43) Veröffentlichungstag der Anmeldung: **30.03.1994**
(21) Anmeldenummer: 93114912.4
(22) Anmeldetag: 16.09.1993
(51) Int. Cl.: A01H 4/00

(54) **Verfahren zur Gewinnung von Protoplasten aus Silybum marianum und entsprechende Pflanzenregeneration**

(30) Priorität: 22.09.1992 DE 4231634
(71) Anmelder: MADAUS Aktiengesellschaft, D-51109 Köln (DE)
(72) Erfinder: Schieder, Otto, Prof. Dr., D-14055 Berlin (DE); Hetz, Erich, D-12167 Berlin (DE); Liersch, Reinhard, Dr., D-53797 Lohmar (DE)

(57) **Zusammenfassung**

Die vorliegende Erfindung betrifft ein Verfahren zur Herstellung genetisch identischer Pflanzen der Gattung *Silybum*, das dadurch gekennzeichnet ist, daß man aus einer Donorpflanze definierten Genotyps Protoplasten herstellt, und anschließend aus den Protoplasten genetisch identische Pflanzen der Gattung *Silybum* regeneriert. Gegenstand der Erfindung sind außerdem die mit Hilfe dieses Verfahrens erhältlichen Protoplasten.

## Beschreibung

Die vorliegende Erfindung betrifft ein Verfahren zur Herstellung genetisch identischer pflanzen der Gattung *Silybum*, sowie Protoplasten von Pflanzen der Gattung Silybum.

Eine der größten Familien von Blütenpflanzen und die einzige Familie der Korbblüterartigen (Asterales) ist die Familie der Korbblütlergewächse (Asteraceae, Compositae). Die Familie der Korbblütlergewächse umfaßt ca. 25 000 Arten von Pflanzen, welche auf etwa 1 100 Gattungen verteilt sind. Die vorliegende Erfindung betrifft Pflanzen der Gattung *Silybum* insbesondere die Art *Silybum marianum* (Mariendistel), welche als Arzneipflanze seit langer Zeit bekannt ist. Diese ist beispielsweise in Europa im Mittelmeerraum angesiedelt. Die Heilpflanze findet insbesondere Anwendung bei der Therapie von Lebererkrankungen. Von besonderem Interesse ist die pharmazeutische Nutzung der Inhaltsstoffe von *Silybum marianum*, wie insbesondere Silymarin. Die Produktivität der Wildpflanze bezüglich der Inhaltstoffe ist jedoch unzureichend. Eine Züchtung ertragreicherer Genotypen ist daher erforderlich. Ziel ist es, den Ertrag pro Flächeneinheit zu steigern, die Erntbarkeit der Pflanze zu erleichtern und die Produktion möglichst auf ein Silibinin zu beschränken.

Die Züchtungsarbeit könnte grundlegend erleichtert werden, wenn es gelänge, für *Silybum* ein Protoplastensystem zu etablieren, woraus Pflanzen in einfacher Weise regeneriert werden könnten. Der entscheidende Vorteil dieser Züchtungsstrategie läge darin, daß aus einzelnen Zellen ganze Pflanzen regeneriert werden können.

Protoplasten sind nackte Einzelzellen, bei denen mit Hilfe spezifischer Enzyme, wie z.B. der Cellulase, der Pectinase, dem Macerozym oder der Driselase, die Zellwände abgebaut, sowie die Einzelzellen aus dem Zellverband gelöst werden. Nach dem Abbau der Zellwände werden die Protoplasten nach außen nur durch das Plasmalemma begrenzt. Die Protoplasten können durch die Wirkung von Pflanzenhormonen, den Phytohormonen, sowie mit speziellen flüssigen oder festen Nährmedien zur Regeneration der Zellwände und zur Zellteilung angeregt werden. Protoplasten können aus verschiedenen Gewebearten gewonnen werden, z.B. aus Blattgewebe (Mesophyll), Blattstielen (Petiolus), etiolierten Keimachsen (Hypokotyle) und Keimblättern (Cotyledonen).

Aus dem derzeitigen Stand der Technik sind keinerlei Protoplastensysteme für mit *Silybum marianum* nahe verwandte Arten aus der Familie der Asteraceen bekannt.

Binding H. et al (Comparative Studies on Protoplast Regeneration in Herbaceous Species of the Dicotyledoneae Class. Z. Pflanzenphysiol.(1981) Bd. 101: 119-130) beschreiben die Pflanzenregeneration aus Protoplasten bei verschiedenen, jedoch mit Silybum nicht eng verwandten Asteraceen, wie zum Beispiel der Sonnenblume. Binding, H., et.al (Protoplast Regeneration to Plants in *Senecio vulgaris* L. Z. Pflanzenphysiol.(1981) Bd. 99: 183-185) beschreiben außerdem die Regeneration von *Senecio vulgaris* L. Pflanzen aus entsprechenden Protoplastenkulturen. Hinweise auf eine mögliche Etablierung eines Protoplastensy-stems für Pflanzen der Gattung *Silybum* sind dem Stand der Technik nicht zu entnehmen.

Aufgabe der vorliegenden Erfindung ist somit die Bereitstellung eines Verfahrens zur Herstellung genetisch identischer Pflanzen der Gattung *Silybum*, insbesondere der Pflanzenart *Silybum marianum*.

Die erfindungsgemäße Aufgabe wird gelöst durch Bereitstellung eines Verfahrens zur Herstellung genetisch identischer Pflanzen der Gattung *Silybum* das dadurch gekennzeichnet ist, daß man a)aus einer Donorpflanze definierten Genotyps Protoplasten herstellt, und b) anschließend aus den Protoplasten genetisch identische Pflanzen der Gattung *Silybum* regeneriert.

In einer bevorzugten Ausführungsform betrifft die Erfindung die Herstellung von Protoplasten von Pflanzen der Gattung *Silybum*, wobei man Laubblätter eines definierten Genotyps der Pflanze zerkleinert, die zerkleinerten Blätter enzymatisch verdaut, um Einzelzellen aus dem Zellverband herauszulösen und um die Zellwände abzubauen und wobei man anschließend die Protoplasten aus dem Verdauungsansatz isoliert. Vorzugsweise entnimmt man die Laubblätter von 12 bis 14 Tage kultivierten Keimlingen. Zur Verdauung verwendet man vorzugsweise ein Enzymgemisch aus Macerozym, Cellulase und Driselase. Am meisten bevorzugt ist gewöhnlich eine Kombination aus etwa 0,02 Gew.-% Mazerozym, 0,5 Gew.-% Cellulose und 0,5 Gew.-% Driselase mit 4,3 mM CaCl₂. Die Verdauung erfolgt unter Lichtausschluß innerhalb von etwa 14 bis 16 Stunden bei einer Temperatur im Bereich von Zimmertemperatur, vorzugsweise bei etwa 26 °C, einem pH von 5 bis 6, vorzugsweise 5,6 bis 5,8 und einer Osmolalität von 650 bis 750 mOsm, vorzugsweise von etwa 700 mOsm.

Vorzugsweise werden die Protoplasten aus dem Verdauungsansatz dadurch isoliert, daß man zunächst größere Gewebestücke, z.B. mit Hilfe von Stahlsieben mit Netzweiten von 250 und 65 µm, abtrennt. Anschließend nimmt man das Rohisolat mehrmals in einer Salzlösung mit 650 bis 750 mOsm, vorzugsweise etwa 700 mOsm, auf und zentrifugiert die Protoplasten bei etwa 110 g ab. Seewasser als Salzlösung ist bei der Reinigung der Protoplasten ein einfaches und nützliches Hilfsmittel, da dessen Osmolalität leicht eingestellt werden kann. Ebenso anwendbar ist aber auch eine NaCl-Lösung der gleichen Osmolalität. Anschließend wird das Pellet über eine 0,6 M Saccharoselösung zentrifugiert, die Protoplastenbanden werden entnommen, mit Salzlösung gewaschen, zentrifugiert und in Protoplastenkulturmedium verdünnt.

Das erfindungsgemäß verwendete Protoplastenkulturmedium (KM I) ist ein modifiziertes Medium auf der Basis eines Mediums nach Kao, K.N. und Michayluk, M.R. (Nutritional requirements for growth of Vicia hajastana cells and protoplasts at a very low population density in liquid media. Planta 126: 105-110 (1975)) (vgl. Beispiel 1).

In diesem Flüssigmedium sind außerdem enthalten:
jeweils etwa 0,2 bis 1,0 mg/l, vorzugsweise jeweils etwa 0,5 mg/l BAP (Benzylaminopurin), NAA (Naphthylessigsäure) und 2,4-D (2,4-Dichloressigsäure).

Die Osmolalität beträgt etwa 650 bis 750 mOsm, vorzugsweise etwa 700 mOsm und der pH-Wert liegt bei etwa 5,5 bis 6,5, vorzugsweise bei etwa 5,8.

In dem erfindungsgemäßen Verfahren zur Protoplastenherstellung verwendet man vorzugsweise Laubblätter von Pflanzen der Spezies *Silybum marianum* und vorzugsweise Pflanzen der Genotypen 1-5/5 Nr. 21-24-1 und 5/6/10-2-1 .

Gemäß einer bevorzugten Ausführungsform der Erfindung werden Pflanzen der Gattung *Silybum* aus einer Protoplastenkultur regeneriert, indem man oben erwähnte Protoplasten von Pflanzen der Gattung *Silybum* in Alginat einbettet, die eingebettenen Protoplasten in einem Phytohormon-haltigen flüssigen Protoplastenkulturmedium unter Ausbildung von Mikrokalli kultiviert, die Mikrokalli unter Auflösung des Alginat-Komplexes isoliert und in Phytohomon-haltigem festem Sproßregenerationsmedium subkultiviert, Einzelkalli der Kultur entnimmt und bis zur Sproßbildung in Sproßregenerationsmedium weiter kultiviert. Die so gebildeten Sproßknospen überführt man dann in hormonfreies Sproßkulturmedium und bewurzelt die Regenerate.

Vorzugsweise nimmt man die Protoplasten zur Alginateinbettung in einer sterilen, etwa 2,1%-igen (w/v) Natriumalginatlösung von etwa 600 bis 700 mOsm, vorzugsweise etwa 640 mOsm, auf und überschichtet damit CaCl₂-haltige Agar-Platten von etwa 650 bis 750 mOsm, vorzugsweise etwa 700 mOsm. Durch Vernetzung des Alginates mit den Calciumionen erfolgt die Einbettung der Protoplasten. Optimale Teilungsraten von über 70% und optimale Bedingungen für die Mikrokallusbildung, erhält man, wenn man bei *Silybum marianum* eine Protoplastendichte von 10⁵ Protoplasten/ml Kulturmedium einstellt (Konditionierungseffekt). Dagegen liegt die Teilungsrate bei nur 35%, wenn eine Protoplastendichte von 5 · 10⁴ gewählt wird.

Die eingebetteten Protoplasten kultiviert man in Protoplastenkulturmedium zunächst etwa 7 Tage bei etwa 26°C im Dunkeln und anschließend bei der gleichen Temperatur und einer Tageslänge von etwa 16 Stunden bei einer Lichtintensität von etwa 3000 Lux. Nach einer gewissen Wachstumsperiode (ca. 45 Tage bei *Silybum marianum*) haben die Mikrokalli eine Dichte und Größe erreicht, die eine Subkultivierung erforderlich machen. Hierzu löst man den gebildeten Alginat-Komplex unter Einwirkung einer Natriumcitratlösung (ca. 20mM, 200 mOsm, pH 5,8) auf, zentrifugiert vorsichtig ab, wäscht mit Protoplastenkulturmedium und transferiert die Mikrokalli auf festes Sproßregenerationsmedium.

Das erfindungsgemäße Sproßregenerationsmedium basiert auf dem MS-Medium von Murashige und Skoog (1962, s.unten). Es liegt in steriler wäßriger Form vor und umfaßt etwa 0,2 bis 0,4 % (w/v), vorzugsweise etwa 0,25% (w/v) Agar, vorzugsweise Gelrite-Agar, etwa 1 bis 3 % (w/v) Saccharose, etwa 1 bis 5 mg/l BAP, 0,25 - 2 mg/l NAA, bis zu etwa 0,625 mg/l Zeatin und bis zu etwa 0,625 mg/l Kinetin.

Nach einer ersten Regenerierungsphase (ca. 25 Tage bei *Silybum marianum*) werden Einzelkalli entnommen und in Sproßregenerationsmedium bei etwa 26°C, einer Tageslänge von etwa 16 Stunden und einer Lichtstärke von etwa 3000 Lux weiter kultiviert, bis sich Sproßregenerate ausbilden. Hierbei wird in geeigneten Intervallen (24 bis 28 Tage bei *Silybum marianum*) subkultiviert. Haben die Sproßregenerate eine Größe von c.a. 2 cm erreicht, so werden sie auf festes Sproßkulturmedium transferiert. Das dabei verwendete Sproßkulturmedium ist ein hormonfreies MS-Medium (nach Murashige, T. und Skoog, F., A revised medium for rapid growth and bioassays with tobacco tissue cultures, Physiol. Plant 15: 473-497 (1962)) mit einem Gehalt von 0,6 bis 1,0 %, vorzugsweise 0,8 % Agar,z.B. Difco-Nobel-Agar, 5 bis 15 ml, vorzugsweise 10 ml, Kokosnußmilch (aus käuflich erworbenen Kokusnüssen gewonnen) je 100 ml Medium und 0,8 bis 1,2 Gew.-%, vorzugsweise 1 Gew.-%, Saccharose und weist einen pH von etwa 5,5 bis 6,5, vorzugsweise 5,8 auf. Die regenerierten Pflänzchen werden bewurzelt und dann in ein Gewächshaus transferiert.

Gegenstand der vorliegenden Erfindung sind außerdem Protoplasten von Pflanzen der Gattung *Silybum*, welche mit Hilfe eines der oben beschriebenen Verfahren erhältlich sind. Gegenstand der vorliegenden Erfindung sind insbesondere Protoplasten von *Silybum marianum* Pflanzen der Genotypen 1-5/5 Nr. 21-24-1 und 5/6/10-2-1.

Die vorliegende Erfindung wird durch die folgenden Beispiele weiter veranschaulicht. Wenn keine gegenteiligen Angabenvorliegen, so erfolgt die pH-Werteinstellung mit einer geeigneten HCl-, NaOH- oder KOH-Lösung.

### Figurenbeschreibung

- Figur 1: zeigt eine schematische Darstellung des erfindungsgemäß angewendeten Verfahrens zur Donorpflanzen-Kultivierung.
- Figur 2: zeigt eine schematische Darstellung des erfindungsgemäß angewendeten Verfahrens zur Protoplastenher - stellung.
- Figur 3: zeigt eine schematische Darstellung des erfindungsgemäß angewendeten Verfahrens zur Sproßregenerierung.

### Beispiel 1

### Herstellung von Protoplasten von Silybum marianum

1. Materialien
a) Genotypen
Grundlage der Protoplastenexperimente sind die *Silybum marianum* Genotypen 5/6/10-2-1 und 1-5/5 Nr. 21-24-1 aus der Einzelpflanzenselektion der Firma Madaus.
b) Kulturmedien
i) Donorpflanzenkulturmedium (hormonfreies MS-Medium)
Hierbei handelt es sich um ein Medium nach Murashige und Skoog (Murashige, T. und Skoog, F. (1962), A revised medium for rapid growth and bioassays with tobacco tissue cultures. Physiol. Plant 15: 473-497) mit 1 % (w/v)Saccharose, einem pH-Wert von 5,8 sowie 1,2% (w/v) Roth-Agar.
ii) Protoplastenkulturmedium (KM I)
KM-Medium (Kao & Michayluk 1975):

| Mineralsalze | mg/l |
|---|---|
| NH₄NO₃ | 600 |
| KNO₃ | 1900 |
| CaCl₂ x 2 H₂O | 600 |
| MgSO₄ x 7 H₂O | 300 |
| KH₂PO₄ | 170 |
| KCl | 300 |
| FeSO₄ x 7 H₂O | 83,4 |
| Na₂EDTA | 104,44 |
| KJ | 0,73 |
| H₃BO₃ | 3 |
| MnSO₄ x H₂O | 10 |
| ZnSO₄ x 7 H₂O | 0,25 |
| CuSO₄ x 5 H₂O | 0,025 |
| CoCl₂ x 6 H₂O | 0,025 |

| Vitamine | mg/l |
|---|---|
| Nicotinamid | 1 |
| Pyridoxin-HCl | 1 |
| Thiamin-HCl | 1 |
| D-Kalziumpantothenat | 1 |
| Folsäure | 0,4 |
| p-Aminobenzoesäure | 0,02 |
| Biotin | 0,01 |
| Cholinchlorid | 1 |
| Riboflavin | 0,2 |
| Ascorbinsäure | 2,0 |
| Vitamin B₁₂ | 0,02 |

| Organische Säuren | mg/l |
|---|---|
| Natriumpyruvat | 20 |
| Zitronensäure | 40 |
| Apfelsäure | 40 |
| Fumarsäure | 40 |

| Zucker | mg/l |
|---|---|
| Fructose | 250 |
| Ribose | 250 |
| Xylose | 250 |
| Mannose | 250 |
| Rhamnose | 250 |
| Cellobiose | 250 |
| Sorbitol | 250 |
| Mannitol | 250 |
| Saccharose | 20 g/l |
| Glucose | 10 g/l |

| Zusätze | mg/l |
|---|---|
| Caseinhydrolysat | 250 |
| Kokosnußwasser | 250 |
| Inositol | 100 |

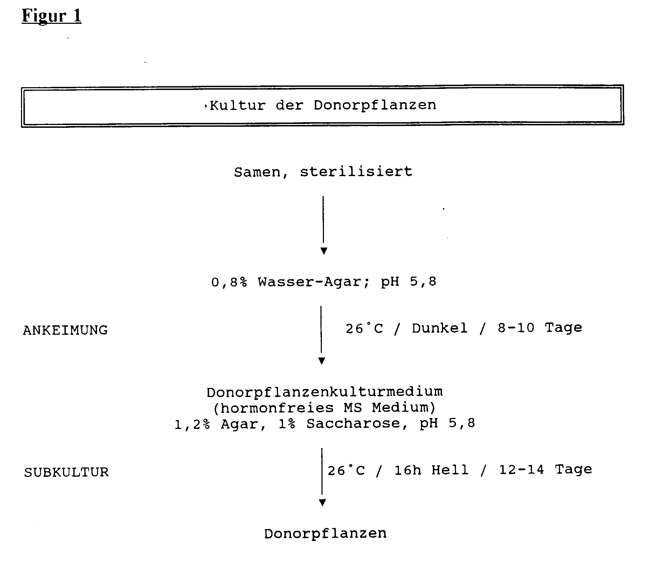

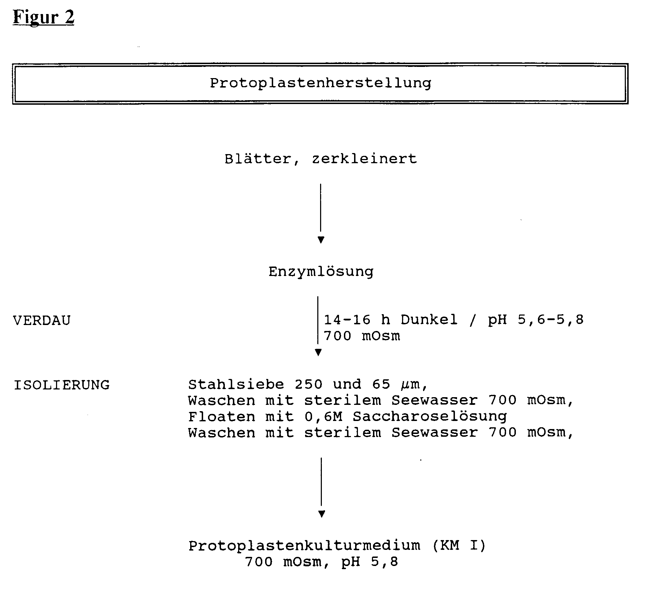

Nach den Vorversuchen mit MI-Medium (nach Li, L. und Kohlenbach,H.W., (1982) Somatic embryogenesis in quite a direct way in cultures of mesophyll protoplasts of Brassica napus L. Plant Cell Rep. 1: 209-211) und KM-Medien (Kao,, K. N. und Michayluk, M.R. (1975) s. oben) fand aufgrund der Ergebnisse in den späteren Ansätzen ausschließlich ein modifiziertes KM-Medium Verwendung. Diesem flüssigen Startmedium sind 0,5 mg/l BAP (Benzylaminopurin), 0,5 mg/l NAA (Naphthylessigsäure) und 0,5 mg/l 2,4-D (2,4-Dichloressigsäure) zugesetzt. Die Osmolalität beträgt 700 mOsm, der pH-Wert 5,8.
2. Kultur der Donorpflanzen
Die Früchte (Samenkörner) werden unter sterilen Bedingungen in Gläsern auf einem Wasseragar (8 g/l Roth-Agar in Leitungswasser mit einem pH- Wert von 5,8) angekeimt. Vorher werden die Früchte in 70% (v/v) Ethanol für 1 min. sowie mit einer 1%-iger Natriumhypochloridlösung (1 % aktives Chlor) für 15 min. sterilisiert und danach dreimal mit sterilem, destillierten Wasser gewaschen. Die Keimung der Früchte erfolgt bei Dunkelheit und 26 °C. Nach 8 - 10 Tagen werden die Keimlinge auf einem hormonfreien MS-Medium (Donorpflanzenkulturmedium) subkultiviert. Die Kultivierung erfolgt im Kulturraum bei einer Tageslänge von 16 Stunden (ca. 3000 Lux) und einer Raumtemperatur von 26°C (vgl. Figur 1)
3. Enzymverdau
12 - 14 Tage nach der Subkultur der Keimlinge werden die ersten Laubblätter von den Pflanzen zum Protoplastieren entnommen. Nach dem Zerkleinern der Blätter wird der Enzymverdau zum Abbau der Zellwände in Rollazellflaschen im Rollomat über eine Dauer von 14 - 16 Stunden durchgeführt. Bei den einzelnen Verdauansätzen werden 2,0 - 2,5 g Blattmaterial eingesetzt und in 50 ml Enzymlösung im Dunkeln bei 26 °C inkubiert.(vgl. Figur 2)
Zusammensetzung der Enzymlösung:
0,02 % Macerozym (Yakult Honsha Co. Ltd. Japan),
0,5 % Cellulase "Onozuka" R 10 (Yakult Honsha Co. Ltd. Japan),
0,5 % Driselase (Sigma)
4,3 mM CaCl₂
in bidestilliertem Wasser.
Der pH-Wert der Enzymlösung wurde auf 5,6 - 5,8, die Osmolalität mit Mannitol auf ca. 700 mOsm eingestellt.
Nach weiteren 10 - 12 Tagen werden ein zweites Mal Laubblätter von den Pflanzen entnommen und protoplastiert. Danach werden die Pflanzen aufgrund zurückgehender Protoplasten-Ausbeute aus der Kultur genommen.
4. Isolierung der Protoplasten
a) Herstellung einer Protoplastensuspension
   Nach der ca. 14 - 16-stündigen Inkubation der Blattstückchen in der Enzymlösung wird die Suspension nacheinander durch zwei miteinander verbundene Stahlsiebe mit Netzweiten von 250 bzw. 65 µm gesiebt, um Gewebestücke abzutrennen. Danach wird mit sterilem Seewasser (Herkunft Sylt), das mit destilliertem Wasser auf ca. 700 mOsm eingestellt ist, 1:1 verdünnt. An Stelle von Seewasser kann auch eine wäßrige NaCl-Lösung der gleichen Osmolalität verwendet werden. Nach Verteilung der Protoplastensuspension auf Zentrifugenröhrchen und 5-minütiger Zentrifugation bei 850 U/min (110 g, Hettich Universal-Zentrifuge) werden die Pellets (die sich aufgrund der Zentrifugation am Boden der Röhrchen befindliche Ablagerung) resuspendiert und in einem Röhrchen zusammengefaßt, mit Seewasser aufgefüllt und erneut 5 min bei 850 U/min (110g) zentrifugiert. Danach wird der Überstand dekantiert.
   Das Floaten der Protoplasten erfolgt anschließend in einer 0,6 M Saccharoselösung in den gleichen Zentrifugenröhrchen durch 10-minütiges Zentrifugieren bei 850 U/min (110 g). Dabei bilden die Protoplasten auf der Saccharoselösung eine deutlich sichtbare Bande aus. Nach Entnahme der Protoplastenbanden mit einer Pasteurpipette werden die Protoplasten in ein Zentrifugenglas überführt. Anschließend wird ein Waschgang mit ca. 10 ml Seewasser und eine 5-minütige Zentrifugation bei 850 U/min (110g) durchgeführt.
   Nach dem Dekantieren des Überstandes werden die Protoplasten mit Protoplastenkulturmedium (KM I) verdünnt (vgl. Figur 2)
b) Einstellen der Protoplastendichte
   Nach der Aufarbeitung wird die Dichte der Protoplasten mit Hilfe einer Zählkammer (nach Neubauer) eingestellt, indem die Zahl der Protoplasten im Zählfeld bestimmt und die Protoplastensuspension entsprechend der gewünschten Dichte mit Protoplastenkulturmedium (KM I) verdünnt wird. Bei *Silybum* ist aufgrund der aus Vorversuchen festgestellten optimalen Teilungsrate von über 70 % eine Dichte von 10⁵ Protoplasten/ml Kulturvolumen einzustellen.

### Beispiel 2

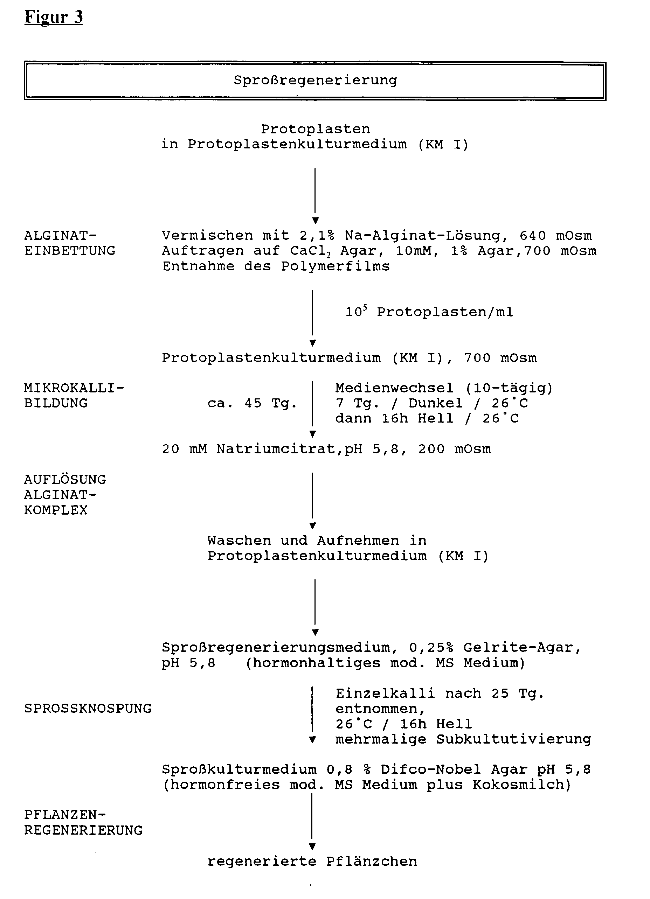

### Regeneration von Silybum marianum aus einer Protoplastenkultur (Figur 3)

1. Medien
a) Protoplastenkulturmedium (KM I) (s.Bsp. 1)
b) Sproßregenerationsmedium
Als Grundmedium dient das MS-Medium nach Murashige und Skoog (s.oben), welches durch unterschiedliche Phytohormonkombinationen sowie zwei verschiedene Saccharosekonzentrationen (1 % bzw. 3 % (w/v)) modifiziert wird. Als Agar wird Gelrite in einer Konzentration von 0,25 % (w/v) verwendet. Alle Medien werden mit doppelt destilliertem Wasser angesetzt, auf einen pH-Wert von 5,8 eingestellt und bei 125 °C für 15 min. autoklaviert. Die verschiedenen Medien enthalten folgende Phytohormonkombinationen:

**Tabelle**

| Sproßregenerationsmedien | | | | |
|---|---|---|---|---|
| Medium | Phytohormone (jeweils mg/l) | | | |
| | BAP | NAA | Zeatin | Kinetin |
| MS 10 | 1 | 0,5 | 0,625 | 0,625 |
| MS 16 | 5 | 2,0 | -- | -- |
| MS 22 | 3 | 0,25 | -- | -- |

c) Sproßkulturmedium
Dieses hormonfreie Medium (basierend auf MS-Medium) wird mit 1 % Saccharose, 100 ml Kokosnußmilch je Liter Medium und 0,8 % (w/v) Difco-Nobel-Agar angesetzt und auf einen pH-Wert von 5,8 eingestellt.
2. Alginateinbettung
Die Einbettung von Protoplasten, hergestellt gemäß Beispiel 1, erfolgt in einer 2,1%-igen (w/v) Natrium-Alginatlösung (Roth GmbH), die mit Mannitol auf 640 mOsm eingestellt wird. Hinzugefügt werden 2,94 g/l CaCl_{2.}
Das schwer lösliche Natriumalginat wird auf einem Rührer über Nacht im Becherglas aufgelöst. Die Sterilisation der Alginatlösung erfolgt durch 15 min. Autoklavieren bei 125°C.
Zu den mit Protoplastenkulturmedium (KM I) auf das Doppelte der gewünschten Kulturdichte (10⁵/ml) eingestellten Protoplastensuspension wird das gleiche Volumen Alginatlösung zugegeben. Die Protoplasten werden vorsichtig mit dem Alginat vermischt und anschließend in einer 10 ml Glaspipette aufgenommen.
Etwa 0,5 ml große Tropfen dieser Protoplasten/Alginat-Mischung werden auf CaCl₂-Agarplatten pipettiert und kräftig geschüttelt, so daß jeder Tropfen eine große Fläche bildete. CaCl₂ bildet mit dem Natriumalginat einen Komplex und bewirkt, daß das Alginat innerhalb etwa 1 Stunde polymerisiert.
Die CaCl₂ Platten werden mit 2,94 g/l CaCl₂ (10mM), 10 g/l Merck-Agar und destilliertem Wasser angesetzt. Die Osmolalität wird mit Mannitol auf 700 mOsm eingestellt. Der Durchmesser der Petrischalen beträgt 9 cm.
Nach der Polymerisation werden die Alginatplättchen mit der Pinzette vorsichtig von dem CaCl₂-Agar abgenommen und in Petrischalen (6 cm Durchmesser) überführt, die 2,5 ml Kulturmedium KM I enthalten.
3. Kultur der Protoplasten
Während der ersten 7 Tage werden die in Alginat eingebetteten Protoplasten in der Dunkelheit im Kulturraum bei ca. 26°C kultiviert. Nach dieser Dunkelphase erfolgte ein Transfer in den Inkubator (Kulturschrank). Die Temperatur wird ebenfalls auf 26°C, die Tageslänge auf 16 Stunden und die Lichtintensität auf ca. 3000 Lux eingestellt.
Der Medienwechsel wird nach folgendem Schema durchgeführt: Nach 10 Tagen wird den 6 cm Petrischalen 2 ml KM I-Medium zugegeben. Dieses Folgemedium enthält die gleichen Phytohormone, die Osmolalität wird aber aufgrund der zwischenzeitlich regenerierten Zellwände bei diesem Medium nicht mehr eingestellt. In Intervallen von 10 Tagen erfolgt ein Medienwechsel, die verbrauchten Medien werden abgesaugt und durch 3 ml frisches KM I-Medium ersetzt.
Die Protoplastenentwicklung kann wie folgt beschrieben werden: In der Kultur regenerieren die Protoplasten innerhalb der ersten drei Tage Zellwände, die ersten Zellteilungen erfolgen nach ca. 3 - 5 Tagen. Mikrokalli, d.h. undifferenzierte Zellverbände, können nach ca. 15 Tagen beobachtet werden.
4. Auflösung des Alginatkomplexes
Nach einer Kulturdauer von etwa 45 Tagen haben sich die Mikrokalli zu einer Größe entwickelt, daß sie aufgrund der Dichte und der beginnenden Phenolbildung auf Festmedien subkultiviert werden müssen. In einem ersten Schritt wird daher der Natriumalginat/CaCl₂-Komplex mit einer 20 mM Natriumcitratlösung aufgelöst. Die Natriumcitratlösung enthält 5,88 g/l Natriumcitrat, die Osmolalität wird mit ca. 3 g Mannitol auf 200 mOsm und der pH-Wert wird auf 5,8 eingestellt. Die Auflösung des Alginates erfolgt in Zentrifugenröhrchen mit 10 ml Natriumcitratlösung. Nach der Auflösung des Alginatkomplexes werden die Mikrokalli bei 500 U/min (ca. 40 g) zentrifugiert, einmal mit 7 ml flüssigem KM I-Medium gewaschen, erneut zentrifugiert und nachfolgend mit 1,5 ml KM I aufgenommen. Anschließend werden die Mikrokalli auf die verschiedenen Festmedien (Sproßregenerationsmedien) transferiert und im Inkubator bei 26 °C und einer Tageslänge von 16 h (ca. 3000 Lux) kultiviert.
5. Subkultur der Mikrokalli
Nach ca. 25 Tagen werden Einzelkalli zur Weiterkultur gepickt, auf den gleichen Medien subkultiviert und unter den gleichen Kulturbedingungen gehalten (26 °C, 16 h Tageslänge, 3000 Lux). Die Dichte je 9 cm Petrischale beträgt am Anfang 58 Einzelkalli und am Ende der Subkulturen 28 Einzelkalli je 9 cm Petrischale. Alle 24 - 28 Tage werden die Kalli subkultiviert. Es werden 116 Kalli je Regenerationsmedium gepickt und subkultiviert, um am Ende 100 Einzelkalli auszuwerten. Bei jedem Ansatz werden vier Subkulturen durchgeführt und danach wird die Sproßregenerationsrate bestimmt.
6. Sproßregeneration (Organogenese)
Die Genotypen 1-5/5 Nr. 21-24-1 und 5/6/10-2-1 zeigen auf den Medien MS 10, MS 16 und MS 22 sowohl bei 1 % als auch bei 3 % Saccharose Sproßregeneration. Die höchste Regenerationsrate wird bei dem Genotyp 1-5/5 Nr. 21-24-1 auf den Medien mit 1 % Saccharose ermittelt. Nach 60 Tagen kann die Sproßregeneration beobachtet werden.
7. Sproßkultur
Die Regenerate (Sproßknospen), die eine Größe von ca. 2 cm erreicht haben, werden in Petrischalen auf sterilem hormonfreiem Sproßkulturmedium kultiviert. Die regenerierten Pflänzchen werden ebenfalls auf hormonfreiem Sproßkulturmedium in 250 ml Kulturgläser bewurzelt und später in das Gewächshaus transferiert.

## Patentansprüche

1. Verfahren zur Herstellung genetisch identischer Pflanzen der Gattung *Silybum*
dadurch gekennzeichnet, daß man
a) aus einer Donorpflanze definierten Genotyps Protoplasten herstellt, und
b) anschließend aus den Protoplasten genetisch identische Pflanzen der Gattung *Silybum* regeneriert.

2. Verfahren nach Anspruch 1, dadurch gekennzeichnet, daß man zur Herstellung von Protoplasten von Pflanzen der Gattung *Silybum*,
a) Laubblätter eines definierten Genotyps der Pflanze zerkleinert,
b) die zerkleinerten Blätter enzymatisch verdaut, um Einzelzellen aus dem Zellverband herauszulösen und um die Zellwände abzubauen, und
c) die Protoplasten aus dem Verdauungsansatz isoliert.

3. Verfahren nach Anspruch 2, dadurch gekennzeichnet, daß die Laubblätter von 12 bis 14 Tage kultivierten Keimlingen entnommen werden.

4. Verfahren nach einem der Ansprüche 2 oder 3, dadurch gekennzeichnet, daß zur Verdauung ein Enzymgemisch aus Macerozym, Cellulase und Driselase verwendet wird.

5. Verfahren nach Anspruch 4, dadurch gekennzeichnet, daß ein Enzymgemisch aus 0,01 bis 0,03 Gew.-%, vorzugsweise 0,02 Gew.-% Macerozym, 0,3 bis 0,7 Gew.-%, vorzugsweise 0,5 Gew.-% Cellulase und 0,3 bis 0,7 Gew.-%, vorzugsweise 0,5 Gew.-% Driselase in 3 bis 5 mM, vorzugsweise 4,3 mM CaCl₂ verwendet wird.

6. Verfahren nach einem der Ansprüche 2 bis 5, dadurch gekennzeichnet, daß die Verdauung unter Lichtausschluß etwa 10 bis 20 Stunden, vorzugsweise etwa 14 bis 16 Stunden, bei etwa 20 bis 30°C, vorzugsweise etwa 26°C, bei einem pH von etwa 5 bis 6,5, vorzugsweise etwa 5,6 bis 5,8 und einer Osmolalität von etwa 680 bis 720 mOsm, vorzugsweise etwa 700 mOsm, erfolgt.

7. Verfahren nach einem der Ansprüche 2 bis 6, dadurch gekennzeichnet, daß man die Protoplasten aus dem Verdauungsansatz isoliert, indem man
i) größere Gewebestücke abtrennt und das Rohisolat mehrmals in einer Salzlösung mit etwa 700 mOsm aufnimmt und die Protoplasten abzentrifugiert,
ii) das Pellet über eine etwa 0,6 M Saccharose-Lösung zentrifugiert, die Protoplastenbanden entnimmt, nochmals in Salzwasser wäscht, zentrifugiert und in Protoplastenkulturmedium verdünnt.

8. Verfahren nach einem der vorhergehenden Ansprüche, dadurch gekennzeichnet, daß man Laubblätter von Pflanzen der Spezies *Silybum marianum* verwendet.

9. Verfahren nach einem der vorhergehenden Ansprüche, dadurch gekennzeichnet, daß man Laubblätter von *Silybum marianum* Pflanzen des Genotyps 1-5/5 Nr.21-24-1 oder 5/6/10-2-1 verwendet.

10. Protoplasten von Pflanzen der Gattung *Silybum* erhältlich nach einem der Ansprüche 2 bis 8.

11. Protoplasten von *Silybum marianum* Pflanzen des Genotyps 1-5/5 Nr.21-24-1 und 5/6/10-2-1, erhältlich nach Anspruch 9.

12. Verfahren nach einem der Ansprüche 1 bis 9, dadurch gekennzeichnet, daß man zur Regeneration von *Silybum* Pflanzen aus einer Protoplastenkultur
a) Protoplasten von Pflanzen der Gattung *Silybum* in Alginat einbettet,
b) die eingebetteten Protoplasten in einem Phytohormonhaltigen Protoplastenkulturmedium unter Ausbildung von Mikrokalli kultiviert, die Mikrokalli isoliert und in Phytohormon-haltigem Sproßregenerationsmedium kultiviert,
c) Einzelkalli der Subkultur entnimmt und bis zur Sproßbildung in Sproßregenerationsmedium subkultiviert,
d) die so gebildeten Sproßknospen in hormonfreies Sproßkulturmedium überführt und die Regenerate bewurzelt.

13. Verfahren nach Anspruch 12, dadurch gekennzeichnet, daß die Protoplasten erhältlich sind nach einem Verfahren gemäß einem der Ansprüche 2 bis 9.

14. Verfahren nach einem der Ansprüche 12 und 13, dadurch gekennzeichnet, daß man in Stufe a) die Protoplasten in einer sterilen, etwa 2,1 %-igen Natriumalginat-Lösung von etwa 640 mOsm aufnimmt und damit CaCl₂ haltige Agarplatten von etwa 700 mOsm überschichtet.

15. Verfahren nach einem der Ansprüche 7 bis 9 oder 12 bis 14, dadurch gekennzeichnet, daß das verwendete Protoplastenkulturmedium die Phytohormone Benzylaminopurin, Naphthylessigsäure und 2,4-Dichloressigsäure umfaßt.

16. Verfahren nach Anspruch 15, dadurch gekennzeichnet, daß jeweils etwa 0,2 bis 1,0 mg/l, vorzugsweise etwa 0,5 mg/l, der einzelnen Phytohormone bei einem pH von etwa 5,5 bis 6,5, vorzugsweise etwa 5,8, und einer Osmolalität von etwa 650 bis 750 mOsm, vorzugsweise etwa 700 mOsm, enthalten sind.

17. Verfahren nach einem der Ansprüche 12 bis 16, dadurch gekennzeichnet, daß man die eingebetteten Protoplasten zunächst im Dunkeln, vorzugsweise etwa 7 Tage bei etwa 26°C, kultiviert und anschließend, vorzugsweise bei etwa 26°C und einer Tageslänge von etwa 16 h und bei einer Lichtintensität von etwa 3000 Lux, weiter kultiviert und dabei in regelmäßigen Abständen einen Mediumswechsel durchführt.

18. Verfahren nach einem der Ansprüche 12 bis 17, dadurch gekennzeichnet, daß das Sproßregenerationsmedium in steriler, wässriger Form vorliegt und etwa
0,25 Gew.-% Agar,
1 bis 3 Gew.-% Saccharose,
1 - 5 mg/l BAP,
0,25 bis 2 mg/l NAA,
bis zu 0,625 mg/l Zeatin und
bis zu 0,625 mg/l Kinetin
umfaßt.

19. Verfahren nach einem der Ansprüche 12 bis 18, dadurch gekennzeichnet, daß das man die Kalli zur Sproßbildung bei etwa 26°C, einer Tageslänge von etwa 16h und bei etwa 3000 Lux in Sproßregenerationsmedium kultiviert.

20. Verfahren nach Anspruch 19, dadurch gekennzeichnet, daß man nach einer ersten Sproßregenerationsphase von etwa 25 Tagen Einzelkalli entnimmt, diese in Sproßregenerationsmedium in Intervallen von 24-28 Tagen solange subkultiviert, bis eine Sproßknospung erfolgt.

21. Verfahren nach einem der Ansprüche 12 bis 20, dadurch gekennzeichnet, daß das Sproßkulturmedium etwa
0,8 bis 1,2 Gew.-%, vorzugsweise etwa 1 Gew.-% Saccharose,
0,6 bis 1,0 Gew.-%, vorzugsweise etwa 0,8 Gew.-% Agar und
5 bis 15ml, vorzugsweise etwa 10 ml Kokosnußmilch / 100 ml Medium
bei einem pH von etwa 5,5 bis 6,5, vorzugsweise etwa 5,8, umfaßt und frei von Phytohormonen ist.

22. Verfahren nach einem der Ansprüche 12 bis 21, dadurch gekennzeichnet, daß die Protoplastendichte bei der Alginateinbettung etwa 10⁵ Protoplasten / ml Ansatz beträgt.
